Europäisches Patentamt

European Patent Office

Office européen des brevets

(19)

(11) Publication number: **0 059 778**

**A1**

(12) **EUROPEAN PATENT APPLICATION**

(21) Application number: **81105771.0**

(22) Date of filing: **22.07.81**

(51) Int. Cl.³: **A 61 B 17/10**

(30) Priority: **09.03.81 US 241865**

(43) Date of publication of application:
**15.09.82 Bulletin 82/37**

(84) Designated Contracting States:
**BE CH DE FR GB IT LI NL SE**

(71) Applicant: **American Cyanamid Company**
**1937 West Main Street P.O. Box 60**
**Stamford Connecticut 06904(US)**

(72) Inventor: **Li, Lehmann K.**
**305 Barlow Road**
**Fairfield Connecticut 06430(US)**

(72) Inventor: **Butter, Reinhart**
**1576 Lafayette Drive**
**Columbus Ohio 43220(US)**

(74) Representative: **Wächtershäuser, Günter, Dr.**
**Gewürzmühlstrasse 5**
**D-8000 München 22(DE)**

(54) **Staple extracting instrument.**

(57) A staple extracting instrument having a first extractor arm (1), the distal end being essentially rounded and containing a slot; a second extractor arm (2), the distal end containing a forming member, and the proximal ends of said first and said second arm containing a partially or completely enclosed first and second handle means (1c, 2c), respectively; and means (3) for connecting said arms, whereby said first and second handle means coordinate to drive said forming member into said slot.

Fig. 1

EP 0 059 778 A1

Croydon Printing Company Ltd.

## STAPLE EXTRACTING INSTRUMENT

This invention relates to a staple extracting instrument. More specifically, this invention relates to a staple extracting instrument for extracting surgical staples from the skin or fascia.

The staple extracting instrument of this invention has advantages over the instruments disclosed in the prior art. One advantage is the distal end of the first extractor arm. The distal end is essentially rounded. This creates a "shovel effect" which enables the distal end of the first extractor arm to be easily placed under the staple to be extracted. Another advantage is that the distal end cannot pick the staple, or the skin or fascia during the placement of the instrument and/or the extracting of the staple.

Another advantage is that the first extractor arm can be manufactured as one piece integral with the distal end. Thus, the possibility weakening and/or bending the distal end, for example by dropping the instrument, is reduced or eliminated. Also, the strength of the distal end is essentially uniform. Thus, the possibility that a portion of the distal end would become weakened and thus cause a malfunction of the instrument during use is reduced or eliminated. This latter advantage may be critical in surgical procedures where time and precision can be and usually are of paramount importance.

Still another advantage is the length from the connecting means to the second handle means which is such that an index, or alternatively, a middle finger can rest on the connecting means. The accuracy in the use of the instrument is thus increased. Yet another advantage is in the first handle means is angled toward said second handle means. The coordinating compressing and expanding motions of the handle means is reduced. With this reduction, the need for a spring means in the expanding motion is eliminated.

Still another advantage of the instrument is in the oval configuration of the first and the second handle means. This configuration tends to distribute the compressing and expanding force over a larger area. Thus the instrument can be more comfortable to the user over a longer period of time.

A staple extracting instrument has been invented. The instrument has a first extractor arm, the distal end being essentially rounded and containing a slot; a second extractor arm, the distal end containing a forming member, and the proximal ends of said first and said second arm containing a partially or completely enclosed first and second handle means, respectively; and means for connecting said arms, whereby said first and second handle means coordinate to drive said forming member into said slot.

Other embodiments include the above described instrument: wherein the length from the connecting to said first handle means is such that an index or, alternatively, a middle finger can rest on said connecting means; wherein said first handle means is angled toward said second handle means; wherein said first handle means has an oval configuration; wherein a thumb can be diagonally placed in said first handle means; wherein said second handle means has an oval configuration; and wherein a ring and a little finger or, alternatively a middle and a ring finger, can be perpendicularly placed in said second handle means.

Still other embodiments include the above described instrument: having a curved portion adjacent to said second handle means; and wherein a middle finger can rest on said curved portion.

- 3 -                    0059778

DESCRIPTION OF THE DRAWINGS

Figures 1 to 6 are right side, left side, back, front, top and bottom views, respectively, of a staple extracting instrument;

Figures 7 and 8 are right side and top views, respectively, of the first extractor arm of Figures 14 to 19; and

Figures 9 and 10 are right side and top views, respectively, of the second extractor arm of Figures 14 to 19.

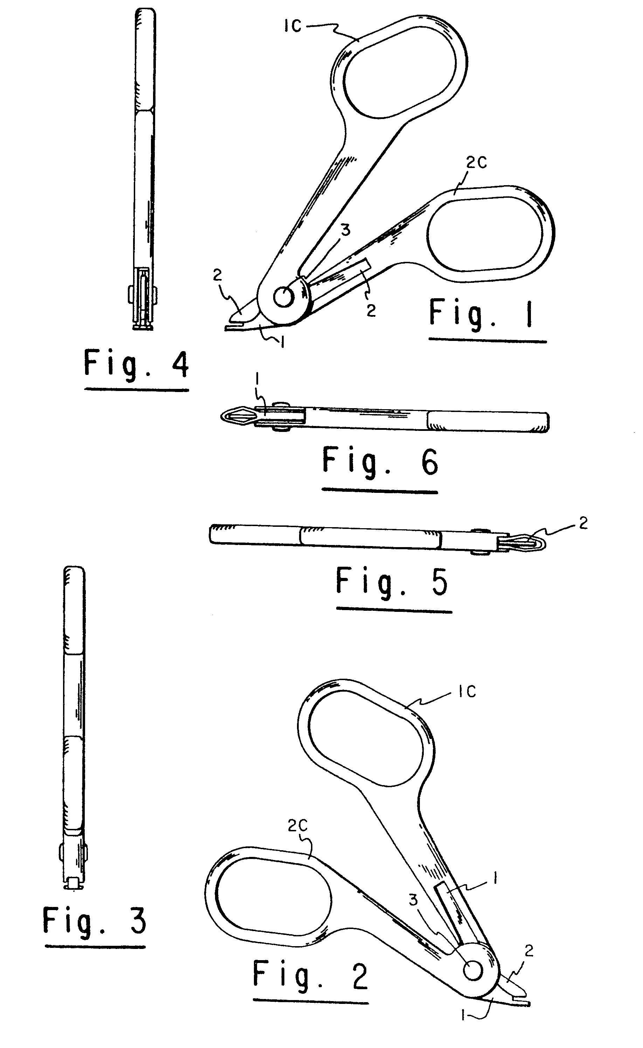

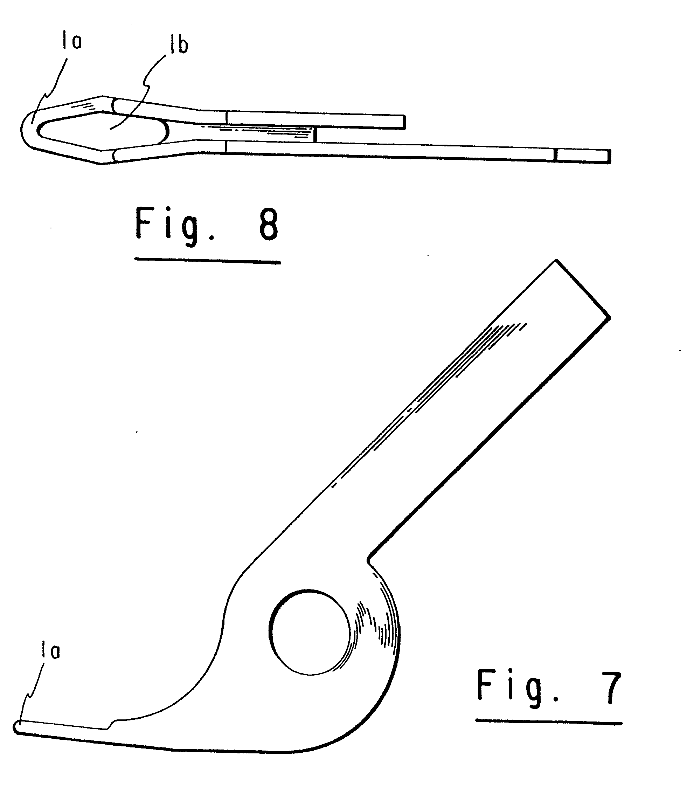

A staple extracting instrument is described. Referring to Figures 1 to 6 , the instrument has a first extractor arm 1. Referring to Figures 7 and 8 , the distal end 1a of the first extractor arm is essentially rounded and contains a slot 1b.

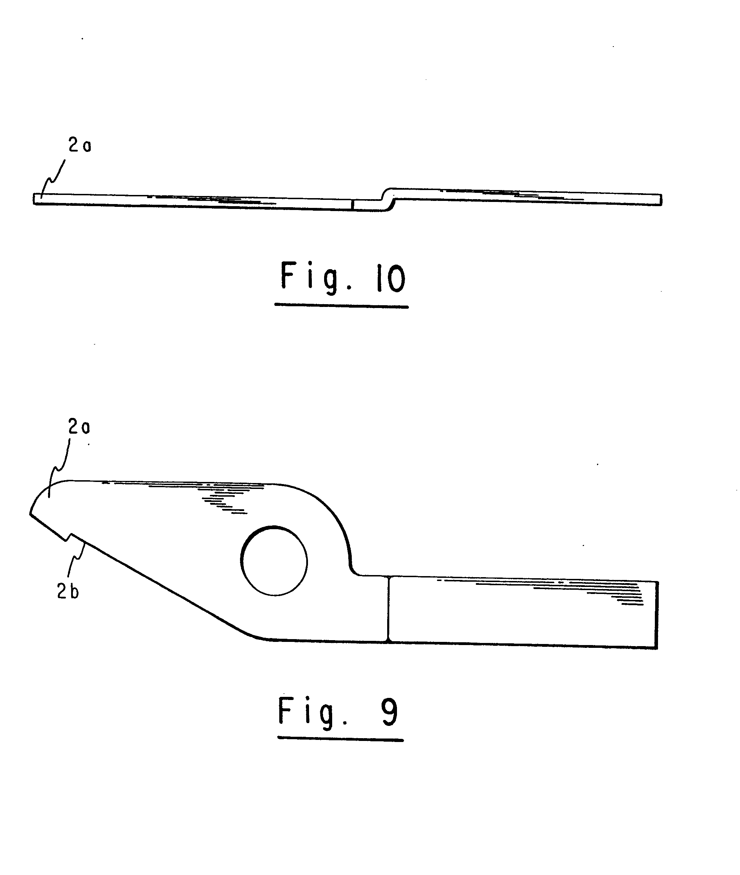

Referring again to Figures 1 to 6 , the instrument has a second extractor arm 2. Referring to Figures 9 and 10 , the distal end 2a of the second extractor arm contains a forming member 2b.

Referring still again to Figures 1 to 6 , the proximal ends of the first and the second extractor arm, 1 and 2 respectively, contain a first and a second handle means, 1c and 2c respectively. Means 3 connect the first and the second extractor arm. The connecting means can be for example a rivet or a nut and bolt.

The staple extracting instrument is used by coordinating the first and/or the second handle means. The distal end 1a of the first extractor arm 1 is placed under a staple. In a coordinating compressing motion, the handle means drive the forming member 2b of the second extractor arm 2 into the slot 1b of the first extractor arm 1. In a coordinating expanding motion, the handle means raise the forming member 2b out of the slot 1b. The staple is bent and is extracted in the compressing motion

Referring to Figures 1 and 2 , the first and the second extractor arm 1 and 2, respectively, are shown as separate pieces from the first and the second handle means 1c and 2c, respectively. It is to be understood however, that either one or both of the extractor arms can be manufactured as one piece integral with the handle means. A suitable material for manufacturing either one or both of the extractor arms and/or the handle means is for example stainless steel. Preferably, the extractor arms are manufactured from a metallic material and the handle means are manufactured from a synthetic polymeric material.

A suitable synthetic polymeric material for manufacturing either one or both of the handle means is for example an acrylomitrile, butadiene and styrene copolymer.

The processes of manufacturing the instrument are known in the prior art. The processes of manufacturing the extractor arms and/or the handle means are also known in the prior art and can be for example die stamping and forming of the metallic material and injection molding of the polymeric material.

It is to be understood that in Figures 1 and 2 the distal ends 1a and 2a respectively, (shown in Figures 7 and 10) of the first and the second extractor arm 1 and 2 respectively, are shown for an understanding of the staple extracting instrument. The distal ends, in relationship to each other, are not necessarily drawn to actual scale. Therefore, the forming member 2b (shown in Figure 9) may be visible in Figure 1 and/or 2 if the distal ends are drawn to actual scale.

Referring to Figures 1 , 2 and 7 to 10 , the first and the second extractor arm 1 and 2, respectively, are shown as separate pieces from the first and the second handle means 1c and 2c, respectively. In this embodiment, the extractor arms contain a tab and the handle means contain a slot. The respective extractor arm tab is then placed in the respective handle means slot. Means 3 which can be for example a rivet or a nut and bolt then connect the first and the second extractor arm. To assist in placing the respective tab into the respective slot, the tab and the slot can be tapered as shown in Figures 1 and 2 .

Also in this embodiment and referring to Figures 8 and 10 , the respective extractor arm tab is offset from the distal end of the first and the second extractor arm. The respective offset tabs provide a greater degree of precision in the manufacture and a greater degree of accuracy in the use of the instrument.

- 6 -

WE CLAIM:

1. A staple extracting instrument having a first extractor arm, the distal end being essentially rounded and containing a slot; a second extractor arm, the distal end containing a forming member, and the proximal ends of said first and said second arm containing a partially or completely enclosed first and second handle means, respectively; and means for connecting said arms, whereby said first and second handle means coordinate to drive said forming member into said slot.

2. An instrument of Claim 1 wherein the length from the connecting means to said first handle means is such that an index finger can rest on said connecting means.

3. An instrument of Claim 1 wherein the length from the connecting means to said first handle means is such that a middle finger can rest on said connecting means.

4. An instrument of Claim 1 or 2 wherein said first handle means is angled toward said second handle means.

5. An instrument of Claim 4 wherein said first handle means has an oval configuration.

6. An instrument of Claim 5 wherein a thumb can be diagonally placed in said first handle means.

7. An instrument of Claim 5 wherein said second handle means has an oval configuration.

8. An instrument of Claim 7 wherein a ring and a little finger can be perpendicularly placed in said second handle means.

9. An instrument of Claim 7 wherein a middle and a ring finger can be perpendicularly placed in said second handle means.

10. An instrument of Claim 7 having a curved portion adjacent to said second handle means.

11. An instrument of Claim 10 wherein a middle finger can rest on said curved portion.

12. A staple extracting instrument package comprising an enclosure and therein an instrument of Claim 1 or 2 or 10.

0059778

1 / 3

Fig. 4

Fig. 1

Fig. 6

Fig. 5

Fig. 3

Fig. 2

la    lb

## Fig. 8

la

## Fig. 7

2a

Fig. 10

2a

2b

Fig. 9

# EUROPEAN SEARCH REPORT

European Patent Office

EP 81 10 5771

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | GB-A-1 172 307 (MALLETS) *Page 1, line 58 - page 2, line 15; figures 1-10* | 1 | A 61 B 17/10 |
| A | | 2,3,5-10 | |
| X | US-A-4 026 520 (ROTHFUSS) *Column 5, lines 5-45; figures 1-6* | 1 | |
| A | | 6,8,9 | |
| A | US-A-3 407 816 (CURUTCHET) *Column 2, lines 20-56; figures 1,5* | 2,9,10 | |

TECHNICAL FIELDS SEARCHED (Int. Cl. 3)

A 61 B

The present search report has been drawn up for all claims

| Place of search | Date of completion of the search | Examiner |
|---|---|---|
| THE HAGUE | 28-05-1982 | BARTLETT S.C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82